# EUROPEAN PATENT APPLICATION

(11) **EP 2 116 238 A1**
(43) Date of publication of application: **11.11.2009**
(21) Application number: 08711919.4
(22) Date of filing: 25.02.2008
(51) Int. Cl.: A61K 31/198, A61K 31/401, A61K 31/4172, A61P 3/02, A61P 3/04, A61P 3/06, A61P 25/22, A23L 1/305, A23L 2/52

(54) **AMINO ACID COMPOSITION**

(30) Priority: 28.02.2007 JP 2007049053
(71) Applicant: Meiji Dairies Corporation, Koto-ku, Tokyo 136-8908 (JP)
(72) Inventor: TSUCHITA, Hiroshi, Odawara-shi Kanagawa 250-0862 (JP); SAITO, Masato, Odawara-shi Kanagawa 250-0862 (JP); SHIN, Kazuo, Odawara-shi Kanagawa 250-0862 (JP); KUBOTA, Yukihiko, Tokyo 136-8908 (JP); YAMADA, Masashi, Moriya-shi Ibaraki 302-0121 (JP); SEKI, Keisuke, Tokyo 136-8908 (JP)
(74) Representative: Behnisch, Werner
(86) International application number: PCT/JP2008/053184
(87) International publication number: WO 2008/105368

(57) **Abstract**

An amino acid composition is provided that can promote fat burning, improve endurance, and reduce fatigue during, for example, marathons, and that can lessen obesity and increase stress resistance.

The amino acid composition comprises 3.0 to 10.0 mass parts of aspartic acid, 2.5 to 9.0 mass parts of threonine, 2.0 to 9.5 mass parts of serine, 12.0 to 40.0 mass parts of glutamic acid, 7.0 to 20.0 mass parts of proline, 0.5 to 4.5 mass parts of glycine, 1.0 to 4.5 mass parts of alanine, 3.0 to 12.0 mass parts of valine, 0.1 to 0.8 mass part of cystine, 1.5 to 5.0 mass parts of methionine, 2.0 to 8.0 mass parts of isoleucine, 5.0 to 15.0 mass parts of leucine, 3.0 to 10.0 mass parts of tyrosine, 3.0 to 10.0 mass parts of phenylalanine, 4.0 to 15.0 mass parts of lysine, 0.8 to 4.0 mass parts of tryptophan, 1.5 to 5.0 mass parts of histidine, and 2.0 to 6.0 mass parts of arginine, wherein the total amount of these amino acids is 100 mass parts.

## Description

### TECHNICAL FIELD

The present invention relates to an amino acid composition that promotes fat burning during sports events which need endurance (for example, cycling), long physical labor and the like, that improves endurance, that reduces fatigue, that reduces obesity and that increases stress resistance.

### BACKGROUND ART

Amino acid compositions effective for reducing fatigue after exercise have been known.
For example, "An amino acid composition comprising in the following molar ratios: 12.6 to 23.4 moles of proline, 8.4 to 15.6 moles of alanine, 13.3 to 24.9 moles of glycine, 8.2 to 15.4 moles of valine, 5.0 to 9.4 moles of threonine, 4.3 to 8.1 moles of leucine, 1.8 to 11.9 moles of histidine, 1.7 to 3.3 moles of serine, 6.0 to 11.2 moles of lysine, 3.1 to 5.9 moles of isoleucine, 2.2 to 10.4 moles of glutamic acid, 2.4 to 4.6 moles of arginine, 2.6 to 5.0 moles of phenylalanine, 4.2 to 7.8 moles of tyrosine, and 1.5 to 2.9 moles of tryptophan." is known (Patent Reference 1).
For another example, a food composition for fatigue recovery, which comprises, as an active ingredient, aspartic acid, and which is effective to take at least 2 g of aspartic acid per day for the adult human, has been known (Patent Reference 2).
For another example, a fatigue recovery agent for the central nervous system, which comprises branched chain amino acids, has been known (Patent Reference 3). In the Patent Reference 3, it is written that a mixture of L-valine, L-leucine, and L-isoleucine as the branched chain amino acids is preferable to be used.
[Patent Reference 1] Japanese Patent No. 3,814,683
[Patent Reference 2] Domestic Re-publication of PCT International Application (International Publication Number: WO 2003/011056)
[Patent Reference 3] Domestic Re-publication of PCT International Application (International Publication Number:WO 2002/034257)

### DISCLOSURE OF THE INVENTION

### THE PROBLEM TO BE SOLVED BY THE INVENTION

In the invention described in Patent Reference 1, it is known that (i) orally administering the amino acid composition in the saliva secreted by hornet larvae (this amino acid composition is known as a vespa amino acid mixture or VAAM) regulates the metabolism of lipid and sugar during exercise and that (ii) an amino acid composition composed of VAAM exhibits a very high correlation with that of the amino acids which are diminishing in the blood because of the fatigue induced by exercise. Based on this knowledge, the invention described in Patent Reference 1 provides a novel amino acid composition by supplementing the VAAM amino acid composition of the above-mentioned (i) with specific amino acids (namely, alanine, valine, histidine, and glutamic acid) , which are the amino acids largely out of the relationship in the above-mentioned (ii), and which indicate the higher reducing ratio of amino acids caused of fatigue. This novel amino acid composition can supply for the reducing amino acids in blood induced by strenuous exercise, can improve exercise performance, and can receive the effects of reducing the fatigue after exercise and the effects of fatigue recovery.
Compared with the lower amount of aspartic acid (no more than 200 mg per single ingestion) presented in nutritionally fortified beverages on the market, the invention described in Patent Reference 2 specifies a large amount of aspartic acid administration (at least 2 g per single ingestion) per day, and by this means quickly improves energy metabolism in the liver and provides a fatigue recovery effect.
Moreover, the invention described in Patent Reference 3 has the effect of specifically contributing to the recovery from and the prevention of the central nervous system fatigue (i.e. brain fatigue) that is caused by, for example, computer work.

However, there is a possibility that amino acid compositions other than the compositions described in Patent References 1 to 3 and the like have various effects such as improving exercise performance.
In particular, if a composition (particularly an orally ingestible liquid composition), which can facilitate fat burning, can improve endurance, and can reduce the fatigue during sporting events necessary for endurance performance (e.g., cycling, rowing, marathons, triathlons) and the fatigue during long physical labor, could be discovered, the composition would be very useful as, for example, a beverage for ingestion prior to or during a sports event, and could provide its effects during, for example, a spurt in the latter half of the sporting events.
Regarding individuals who have a constitution to get fat easily, if an amino acid composition having the effect of preventing the intracellular accumulation of fat granules were to exist, the ingestion of such an amino acid composition could be expected to have an anti-obesity effect (i.e. lessening, inhibiting, or preventing obesity).
In this regard, lots of the amino acid compositions written in the prior arts, as described in Patent References 1 to 3, quite frequently have the characteristics of recovering fatigue rather than improving endurance performance and the like as their main objective.
An object of the present invention is therefore to provide an amino acid composition that can facilitate fat burning, improve endurance, and prevent or reduce or recover fatigue, during sporting events which need endurance such as cycling, and during long physical labor; that can lessen, inhibit, or prevent obesity in individuals to get fat easily; and that can improve stress resistance in, for example, office workers.

### MEANS FOR SOLVING THE PROBLEM

As a result of extensive investigations directed to solving the problem described above, the present inventors discovered that an amino acid composition having approximately the same as the amino acid composition that constitutes casein, which is the protein presented in, for example, cow's milk, provides the effects of promoting fat burning, improving endurance, and preventing or reducing or recovering fatigue, during sporting events which need endurance such as cycling, and during long physical labor, and can lessen obesity in the individuals, who have a constitution to get fat easily, and the like. The present invention has been achieved based on this discovery.
Thus, the present invention provides the following [1] to [3].
[1] An amino acid composition comprising the following amino acids in the following amounts wherein the total quantity of the following 18 amino acids is 100 mass parts.

| | |
|---|---|
| aspartic acid | 3.0 to 10.0 mass parts |
| threonine | 2.5 to 9.0 mass parts |
| serine | 2.0 to 9.5 mass parts |
| glutamic acid | 12.0 to 40.0 mass parts |
| proline | 7.0 to 20.0 mass parts |
| glycine | 0.5 to 4.5 mass parts |
| alanine | 1.0 to 4.5 mass parts |
| valine | 3.0 to 12.0 mass parts |
| cystine | 0.1 to 0.8 mass part |
| methionine | 1.5 to 5.0 mass parts |
| isoleucine | 2.0 to 8.0 mass parts |
| leucine | 5.0 to 15.0 mass parts |
| tyrosine | 3.0 to 10.0 mass parts |
| phenylalanine | 3.0 to 10.0 mass parts |
| lysine | 4.0 to 15.0 mass parts |
| tryptophan | 0.8 to 4.0 mass parts |
| histidine | 1.5 to 5.0 mass parts |
| arginine | 2.0 to 6.0 mass parts |

[2] The amino acid composition according to [1], wherein the amino acid composition is for oral ingestion.
[3] The amino acid composition according to [1] or [2], wherein the amino acid composition is used to obtain at least one effect selected from promoting fat burning during exercise, improving endurance during exercise, preventing or reducing fatigue induced by exercise or supporting recovery from fatigue induced by exercise, obesity resistance, and stress resistance.

In a sporting event which needs endurance (e.g., cycling, rowing, marathon, triathlon, and so forth) or long physical labor in factories, the amino acid composition of the present invention can bring about a larger work output compared with that in the absence of ingestion of the amino acid composition of the present invention, even when a switch is made from a normal exercise load to a strenuous exercise load. Accordingly, a significant effect can be expected during, for example, a spurt in the latter half of a sporting event.
The amino acid composition of the present invention can also prevent or lessen fatigue induced by exercise or can support recovery from fatigue induced by exercise.
The amino acid composition of the present invention can also promote fat burning in the individual who has ingested the amino acid composition of the present invention.
The amino acid composition of the present invention can also lessen, inhibit, or prevent obesity, for example, in individuals who have a constitution to get fat easily.
The amino acid composition of the present invention can also expand the capacity to resist stress.
The amino acid composition of the present invention can thus provide at least one effect selected from promoting fat burning during exercise, improving endurance during exercise, preventing or reducing fatigue induced by exercise or supporting recovery from fatigue induced by exercise, preventing obesity, and resistance to stress. Particularly in a sporting event which needs endurance, long physical labor, or the like, the amino acid composition of the present invention can provide a promotion of fat burning and an improvement in endurance as well as the ability to prevent or lessen fatigue or support a fatigue recovery. With regard to individuals whose purpose is the resistance to obesity, the amino acid composition of the present invention can provide both of a reducing, inhibition, or prevention of obesity, and a prevention of, reducing of, or recovery from fatigue induced by exercise for losing weight. The amino acid composition of the present invention can also provide stress resistance to individuals who are engaged in high-stress occupations and the like.
Thus, the amino acid composition of the present invention is useful, for example, to athletes seeking to improve their competitive results, to individuals who are engaged in long physical labor, to individuals seeking an anti-obesity effect, and to individuals who are engaged in high-stress occupations.

### BRIEF DESCRIPTION OF THE DRAWINGS

Figure 1 is a protocol of the time schedule in experiments of improvements in endurance(Example 1, and Comparative Examples 1 to 2);
Figure 2 is a graph that shows the level of the activity of releasing free-fatty acid in the mouse fibroblast;
Figure 3 is a graph that shows the results of the period of swimming at the first trial;
Figure 4 is a graph that shows the time course of the period of swimming at each trial over three weeks;
Figure 5 is a graph that shows the time course of the concentration of 3-hydroxybutyric acid in blood; and
Figure 6 is a graph that shows the results of experiments for evaluating resistance to fatigue in mice.

### BEST MODE FOR CARRYING OUT THE INVENTION

The amino acid composition of the present invention comprises the following amino acids in the following amounts (wherein the total quantity of the following 18 amino acids is 100 mass parts).
The mass proportion for aspartic acid (Asp) is 3.0 to 10.0 mass parts, preferably 4.0 to 9.0 mass parts, more preferably 5.0 to 8.0 mass parts, and particularly preferably 6.0 to 7.0 mass parts.
The mass proportion for threonine (Thr) is 2.5 to 9.0 mass parts, preferably 3.0 to 7.5 mass parts, more preferably 3.5 to 5.5 mass parts, and particularly preferably 3.8 to 4.8 mass parts.
The mass proportion for serine (Ser) is 2.0 to 9.5 mass parts, preferably 3.0 to 8.0 mass parts, more preferably 4.5 to 6.5 mass parts, and particularly preferably 5.0 to 6.0 mass parts.
The mass proportion for glutamic acid (Glu) is 12.0 to 40.0 mass parts, preferably 14.0 to 32.0 mass parts, more preferably 16.0 to 25.0 mass parts, and particularly preferably 18.0 to 23.0 mass parts.
The mass proportion for proline (Pro) is 7.0 to 20.0 mass parts, preferably 8.0 to 17.0 mass parts, more preferably 9.0 to 13.0 mass parts, and particularly preferably 9.5 to 11.5 mass parts.

The mass proportion for glycine (Gly) is 0.5 to 4.5 mass parts, preferably 0.8 to 3.5 mass parts, more preferably 1.2 to 2.5 mass parts, and particularly preferably 1.4 to 2.2 mass parts.
The mass proportion for alanine (Ala) is 1.0 to 4.5 mass parts, preferably 1.5 to 4.0 mass parts, more preferably 2.0 to 3.5 mass parts, and particularly preferably 2.3 to 3.3 mass parts.
The mass proportion for valine (Val) is 3.0 to 12.0 mass parts, preferably 4.0 to 10.0 mass parts, more preferably 5.0 to 8.0 mass parts, and particularly preferably 5.5 to 7.0 mass parts.
The mass proportion for cystine (Cys) is 0.1 to 0.8 mass part, preferably 0.1 to 0.6 mass part, more preferably 0.2 to 0.5 mass part, and particularly preferably 0.2 to 0.4 mass part.
The mass proportion for methionine (Met) is 1.5 to 5.0 mass parts, preferably 1.8 to 4.0 mass parts, more preferably 2.1 to 3.0 mass parts, and particularly preferably 2.3 to 2.9 mass parts.
The mass proportion for isoleucine (Ile) is 2.0 to 8.0 mass parts, preferably 3.0 to 7.0 mass parts, more preferably 4.0 to 6.0 mass parts, and particularly preferably 4.5 to 5.4 mass parts.

The mass proportion for leucine (Leu) is 5.0 to 15.0 mass parts, preferably 6.0 to 12.0 mass parts, more preferably 7.5 to 10.0 mass parts, and particularly preferably 8.0 to 9.5 mass parts.
The mass proportion for tyrosine (Tyr) is 3.0 to 10.0 mass parts, preferably 4.0 to 8.0 mass parts, more preferably 4.5 to 6.5 mass parts, and particularly preferably 4.8 to 5.8 mass parts.
The mass proportion for phenylalanine (Phe) is 3.0 to 10.0 mass parts, preferably 3.5 to 8.0 mass parts, more preferably 4.0 to 6.0 mass parts, and particularly preferably 4.2 to 5.5 mass parts.
The mass proportion for lysine (Lys) is 4.0 to 15.0 mass parts, preferably 5.0 to 11.0 mass parts, more preferably 6.0 to 9.0 mass parts, and particularly preferably 7.0 to 8.0 mass parts.
The mass proportion for tryptophan (Trp) is 0.8 to 4.0 mass parts, preferably 1.0 to 3.0 mass parts, more preferably 1.1 to 2.0 mass parts, and particularly preferably 1.2 to 1.8 mass parts.
The mass proportion for histidine (His) is 1.5 to 5.0 mass parts, preferably 1.8 to 4.0 mass parts, more preferably 2.1 to 3.2 mass parts, and particularly preferably 2.3 to 3.0 mass parts.
The mass proportion for arginine (Arg) is 2.0 to 6.0 mass parts, preferably 2.5 to 5.0 mass parts, more preferably 3.0 to 4.0 mass parts, and particularly preferably 3.1 to 3.8 mass parts.
For lysine, histidine, and arginine, the amount of each amino acid refers to the state in which a plus charge is present (for example, for lysine the terminal of the side chain is -NH₃⁺). For aspartic acid and glutamic acid, the amount of each amino acid refers to the state in which a minus charge is present (i.e. the state in which the amino acid does not include a cation such as sodium).
The cystine used in the present invention may be present in the form of cysteine as provided by the partial or complete reduction of the cystine. In this case, the amount of the cysteine is converted to the amount of cystine, and makes up all or a portion of "the amount of cystine" defined in the present invention.
The amino acids used in the present invention may also be present as a salt (e.g., the sodium salt, the hydrochloride, and so forth) or as a derivative. However, the "amount of amino acid" defined in the present invention, as already noted above, does not include the cation (e.g., sodium and so forth) or the anion (e.g., the chloride ion and so forth).

The amino acids used in the present invention preferably take the form composed in protein (i.e. the L form; the natural form).
The amino acids used in the present invention may include the form of a peptide in which from 2 to 5 amino acids are bonded. In such a case, the previously described amount for each of the amino acids includes the quantity of the peptide. However, the amino acid composition of the present invention preferably does not contain peptides.
The amino acid composition of the present invention may also contain the following components as optional components: vitamin C, vitamin B₁, vitamin B₂, vitamin B₆, sugar, fructose, sucrose, trehalose, citric acid, sucralose, pectin, baking soda (i.e. sodium bicarbonate), stevia, flavorant, other amino acids (for example, glutamine ,(Gln), asparagine (Asn)), and so forth.
An example of a method of preparing the amino acid composition of the present invention is to mix the amino acids, which can be purchased commercially, in the proportions mentioned above.
The amino acid composition of the present invention may be used in the form of a liquid (for example, an aqueous solution), solid (powder, granule, tablet, capsule, and so forth), or semisolid (jelly and the like), but desirably takes the form of a liquid or semisolid from the perspective of ease of ingestion. For the liquid form, intake or ingestion may be carried out by oral administration, injection, rectal administration, and so forth. The solid and semisolid forms may be ingested by oral administration.
Liquid and semisolid are the particularly preferred forms of use in the present invention. These forms have the advantages of easy intake and easy adjustment of the daily amount of intake within an appropriate numerical value range.
The amount of intake of the amino acid composition of the present invention, as the mass of the total quantity of the 18 amino acids specified for the present invention, is preferably 0.5 to 20 g per ingestion, more preferably 1 to 15 g per ingestion, and particularly preferably 3 to 10 g per ingestion. When this amount is less than 0.5 g, the effects of the invention are not satisfactorily obtained. When this amount is more than 20 g, excessively huge amounts of water are required to satisfactorily dissolve the amino acids in the case of ingestion of the liquid form. And those huge amounts of water cause a large volume for the total liquid form and thereby, for example, cause to be difficult to ingest.
The times of daily ingestion of the amino acid composition of the present invention is not particularly limited, but is ordinarily 1 to 5 times.
The amino acid composition of the present invention can be ingested in the form of, for example, drugs, quasi-drugs, soft drinks, carbonated drinks, food products (in the form of solid or semisolid), and so forth.

### EXAMPLES

### [Experiments in Athletes (Example 1 and Comparative Examples 1 and 2)]

The object of these experiments was 12 athletes (6 males, 6 females) of sports which need endurance (for example, cycling, rowing, or triathlon). The following (a)-(c) were evaluated for a beverage containing the amino acid composition of the present invention, water, and flavor (Example 1), a beverage containing glucose, water, and flavor (Comparative Example 1), and a beverage containing water and flavor (Comparative Example 2): (a) work done during full exercise, (b) total amount of fat oxidation during steady-state submaximal exercise, (c) changes of plasma glucagon concentration and free-fatty acid, and (d) change of blood lactate concentration.

### [A. Beverage preparation]

### (a) Preparation of the beverage according to the present invention (Example 1)

A beverage having a weight of 380 g was prepared to mix 1.7 g of flavor, 6 g of an amino acid composition with following mass parts, wherein the total amount of the following 18 amino acids was 100 mass parts, and water.

| (amino acid) | (amount of incorporation) | (as mol%) |
|---|---|---|
| aspartic acid | 6.6 mass parts | 6.5 mol% |
| threonine | 4.3 mass parts | 4.8 mol% |
| serine | 5.5 mass parts | 7.0 mol% |
| glutamic acid | 20.3 mass parts | 18.3 mol% |
| proline | 10.6 mass parts | 12.3 mol% |
| glycine | 1.8 mass parts | 3.2 mol% |
| alanine | 2.8 mass parts | 4.2 mol% |
| valine | 6.2 mass parts | 7.0 mol% |
| cystine | 0.3 mass part | 0.2 mol% |
| methionine | 2.6 mass parts | 2.3 mol% |
| isoleucine | 5.0 mass parts | 5.0 mol% |
| leucine | 8.8 mass parts | 8.9 mol% |
| tyrosine | 5.4 mass parts | 3.9 mol% |
| phenylalanine | 4.8 mass parts | 3.9 mol% |
| lysine | 7.5 mass parts | 6.8 mol% |
| tryptophan | 1.5 mass parts | 1.0 mol% |
| histidine | 2.7 mass parts | 2.3 mol% |
| arginine | 3.4 mass parts | 2.6 mol% |

### (b) Preparation of the comparative beverage (Comparative Example 1)

A beverage having a weight of 380 g was prepared to mix 6 g of glucose, 1.7 g of flavor, and water.

### (c) Preparation of the comparative beverage (Comparative Example 2)

A beverage having a weight of 380 g was prepared to mix 1.7 g of flavor and water.

### [B. Procedure of experiments]

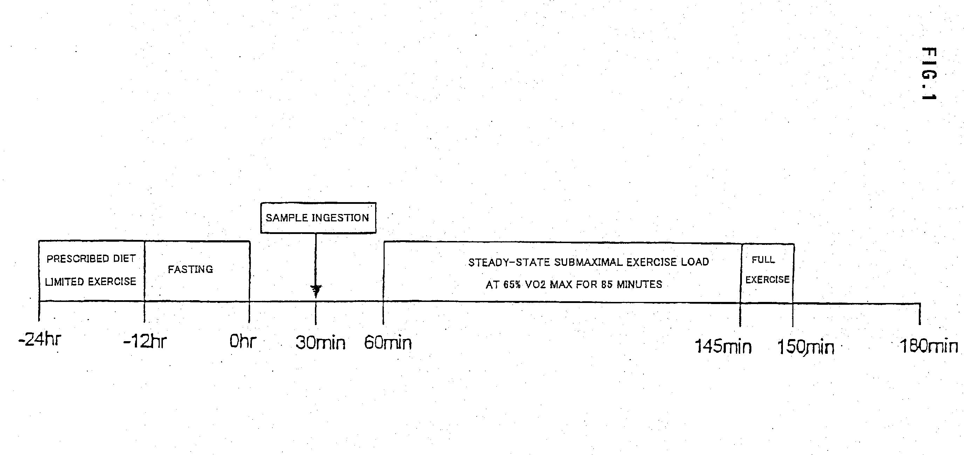

The test used a 3-way double-blind crossover design. The 12 athletes were divided into 6 groups. Each of the groups, which contains 2 athletes, is used in each of the following six kinds of experimental sequential procedures with 3 kinds of beverages (i.e. Example 1 and Comparative Examples 1 and 2).
(1) sequence of Example 1, Comparative Example 1, Comparative Example 2
(2) sequence of Example 1, Comparative Example 2, Comparative Example 1
(3) sequence of Comparative Example 1, Example 1, Comparative Example 2
(4) sequence of Comparative Example 1, Comparative Example 2, Example 1
(5) sequence of Comparative Example 2, Example 1, Comparative Example 1
(6) sequence of Comparative Example 2, Comparative Example 1, Example 1
The protocol for the test was as shown in Figure 1. In Figure 1, the time, when the fasting was finished, was designated as the base time (i.e. time 0). A prescribed diet and limited exercise had been done during the period from 24 hours before the base time to 12 hours before the base time; fasting was carried out in the period of from 12 hours before the base time to time 0; and the beverage (i.e. Example 1, Comparative Example 1, or Comparative Example 2) was orally ingested at 30 minutes after the finish of the fasting. After the lapse of 30 minutes after ingesting the beverage (designated as the "sample" in Figure 1), exercise with a stationary training bicycle was performed for 85 minutes at a work load equivalent to 65% of the maximum oxygen intake (referred to below as steady-state submaximal exercise), and, after finishing steady-state submaximal exercise, as much work as possible was completed in 5 minutes on the stationary training bicycle.

### [C. Evaluation of the effects of the beverage]

### (a) Amount of work during full exercise

The amount of work during the 5 minutes of full exercise (average value of the 12 participants) was 86.1 kJ in Example 1, 83.3 kJ in Comparative Example 1, and 83.6 kJ in Comparative Example 2.
Larger values for the amount of work are indicative of better endurance.
The 86.1 kJ value in Example 1 was significantly higher (p < 0.05) than the value in Comparative Example 1 (83.3 kJ) and the value in Comparative Example 2 (83.6 kJ).
The statistical analysis for the each amount of work (i.e. endurance) used paired t-testing.

### (b) Total amount of fat oxidation during steady-state submaximal exercise (amount of burning fat in a body)

The total amount of fat oxidation during 85-minute steady-state submaximal exercise was 56.7 g in Example 1, 49.3 g in Comparative Example 1, and 45.9 g in Comparative Example 2.
Larger values for the total amount of fat oxidation indicate a greater inhibition of glycolysis, and as a result a smaller amount of utilizing glycogen. In general, the control to utilize as small amount of glycogen as possible and efficient burning of body fat enable exercise to be continued for a long time without fatigue.
The 56.7 g in Example 1 was significantly higher (p < 0.01) than the value in Comparative Example 1 (49.3 g) and the value in Comparative Example 2 (45.9 g).
The statistical analysis for the each amount of fat oxidation is used paired t-testing.

### (c) Measurement of plasma glucagon concentration and free-fatty acid concentration

Plasma glucagon concentration and free-fatty acid concentration were also measured. From the result of measurement, a tendency was seen, throughout the experimental period from beverage intake to post-full exercise, that the sequence from higher concentration to lower concentration was Example 1, Comparative Example 1 and Comparative Example 2 (particularly during sustained exercise). This tendency was the same as that for the total amount of fat oxidation. Glucagon activates hormone-sensitive lipase and acts to promote lipolysis and increase the amount of free-fatty acid. These results of measurement of the plasma glucagon concentration and the plasma free-fatty acid concentration are correlated with the results of measurement of the total amount of fat oxidation during the sustained exercise period.
As the statistical analysis for the each measuring amount of plasma glucagons concentration and free-fatty acid concentration, "repeated analysis of variance" (repeated-ANOVA) is used.

### (d) Change in blood lactic acid concentration during as much exercise as possible

The concentration of lactic acid after 5-minute full exercise was 9.8 minol/L in Example 1, 8.8 mmol/L in Comparative Example 1, and 8.0 mmol/L in Comparative Example 2.
The concentration of lactic acid immediately before full exercise (i.e., after the sustained exercise period) was 1.4 mmol/L in Example 1, 1.5 mmol/L in Comparative Example 1, and 1.6 mmol/L in Comparative Example 2.
Larger lactic acid concentration values after full exercise indicate that larger amount of the carbohydrate, which saved during sustained exercise, is consumed and converted to lactic acid during full exercise. Thus, larger lactic acid concentration values after full exercise indicate a larger volume of work during the last-spurt full exercise interval and as a consequence indicate an improvement of endurance.
As the statistical analysis for the each measuring amount of blood lactic acid concentration, "repeated analysis of variance" (repeated-ANOVA) is used.
These results from the evaluation of the (a) work done during full exercise, (b) total amount of fat oxidation during steady-state submaximal exercise, (c) changes of plasma glucagon concentration and free-fatty acid, and (d) change of blood lactic acid concentration, indicate that significant effects are obtained by the ingestion of beverages containing the amino acid composition of the present invention (Example 1) for the promotion of fat burning, improvement in endurance, prevention or lessening of or recovery from fatigue, anti-obesity, and so forth in comparison with the ingestion of beverages not containing the amino acid composition of the present invention (Comparative Examples 1 and 2). Effectiveness in terms of stress resistance may also be predicted.

### [In vitro Experiments (Example 2 and Comparative Examples 3 to 5)]

Experiments were carried out for investigating whether the amino acid composition of the present invention is active in inhibiting the intracellular accumulation of fat granules (specifically, the ability to inhibit the induction of the differentiation of preadipocytes).
A high activity in this regard indicates a better inhibition of intracellular fat granule accumulation and the presence of an anti-obesity effect. It may also indicate a better utilization of carbohydrate and lipid as energy sources during exercise, an improvement of endurance in exercise, and a lessening in fatigue.

### [Theory of Experiment]

The theory of the experiment is as follows.
Mouse fibroblasts (3T3-LI cells) efficiently differentiate into adipocytes in the presence of dexamethasone (DEX), isobutylmethylxanthine (IBMX), and insulin. It is known that the addition of "Wortmannin", which is a substance that inhibits the induction of differentiation to adipocytes, inhibits differentiation into adipocytes. This inhibitory effect can be evaluated by measuring the GPDH activity.
The GPDH activity is quantitated in terms of units where 1 unit equals 1 nmol of NADH which is oxidized for one minute in 1 mL of the culture medium for inducing the differentiation of mouse fibroblasts (3T3-LI cells) into adipocytes. A smaller value of units indicates a greater inhibitory effect on differentiation and a greater inhibition of intracellular fat granule accumulation.

### [Example 2]

### (a) Preparation of the culture medium

The culture medium was obtained by dissolving the same amino acid composition of the present invention as that in Example 1 into D-MEM medium (i.e. serum-containing liquid medium) to provide a concentration of 1 mass%. Almost of the all amount of the amino acid composition was dissolved well by ultrasonication.
The obtained culture medium was then adjusted to pH8.2 by adding 1 N sodium hydroxide solution. After this pH adjustment, the culture medium was sterilized by filtration to yield a culture medium used for evaluation.

### (b) Procedure for inducing the differentiation of mouse fibroblasts (3T3-L1 cells) into adipocytes

Culture medium which contains mouse fibroblasts (3T3-L1 cells) was treated with trypsin and then was subjected to centrifugal separation at 1,000 rpm for 3 minutes at 4°C. The solid part of component by centrifugal separation, which contains the cultured cells, was dispensed into a multiwell plate (number of wells: 24) at a number of cell as 4.2 × 10⁴ per well to produce the test plate. This test plate was cultivated for 72 hours at 37°C in the atmosphere containing 5% of carbon dioxide. After the cultivation, the culture medium for evaluation, which has already been prepared in the above (a), was dispensed into each well. This was followed by incubating for 30 minutes at 37°C in the atmosphere containing 5% of carbon dioxide. Dexamethasone, isobutylmethylxanthine, and insulin were then added to each well of the evaluation plate to obtain final concentrations of 20 µM, 10 µM, and 1.7 µM respectively. After 48 hours after this addition, the liquid fraction (i.e. culture medium used for evaluation) in each cell was removed, and fresh culture medium for evaluation and insulin were dispensed to each well to obtain a final concentration of 1.7 µM of insulin. This was followed by cultivating for 12 days. The above-mentioned procedure (i.e. replacement of the culture medium used for evaluation and addition of insulin at each well) was repeated at every 3 day over the total 12-day cultivating period.

### (c) Method of measuring the GPDH activity

A solid fraction was obtained by removing the liquid fraction with centrifugal separation from the culture medium yielded above (b). Washing was done twice using 2 mL of ice-cooled phosphate-buffered saline (PBS, phosphate buffer). To the washed solid fraction (i.e. cells) was added 600 µL of Tris buffer (i.e. mixture of 50 mM Tris-HCl buffer pH 7.5, 1 mM EDTA, and 1 mM β-mercaptoethanol). After that, the solid fraction (i.e. cells) was suspended by using a cell scraper to provide a cell suspension. This cell suspension was then placed in a 1.5 mL plastic tube.
The cell suspension was ultrasonicated in order to break the cell membranes and was thereafter subjected to centrifugal separation for 15 minutes at 15,000 rpm to obtain a liquid fraction (liquid temperature: 4°C), which was a cell extract.
The protein concentration of the cell extract was quantitated by using Bradford's dye-binding method, and the concentration of the protein in the liquid was then adjusted to a prescribed value (21 µg/assay) by adding the same Tris buffer as described above.

The GPDH activity of this cell extract, which has been adjusted to the prescribed concentration of protein, was measured. The method of measuring GPDH activity is as follows: at first, an ice-cooled reaction solution (i.e. a liquid mixture of 100 mM of triethanolamine-HCl pH 7.5, 0.12 mM of NADH, and 0:1 mM of β-meraaptoethanol) was added to the cell extract to adjust the total volume to 950 µL; the solution was kept for 5 minutes at 25°C; 50 µL of a reaction starting solution (i.e. 4 mM of dihydroxyacetone phosphate) was added to bring the reaction solution as total volume to 1 mL; and the one-minute change in optimal absorbance at 340nm wavelength was measured on the resulting solution at 25°C.
The GPDH activity is represented by using unit. 1 unit means the amount of enzyme that oxidizes 1 nmol of NADH in one minute.
The result of the measurement was 3 units.

### [Comparative Example 3]

A culture medium used for evaluation was obtained by using the same procedure as in Example 2 except that the amino acid composition of Example 2 was replaced by an amino acid composition prepared by mixing the branched-chain amino acids of valine, leucine, and isoleucine with the mass ratio of 1 : 2 : 1. By using the resulting culture medium used for evaluation, the GPDH activity was measured as in Example 2.
The result of the measurement was 168 units.

### [Comparative Example 4]

An evaluation culture medium was obtained in the same way as in Example 2 except that in this case the culture medium was obtained by dissolving "Wortmannin", which is a substance that inhibits the induction of differentiation to the adipocyte, in the D-MEM medium with the concentration of 10 µM. By using the resulting culture medium for evaluation, the GPDH activity was measured in the same way as in Example 2.
The result of the measurement was 47 units.

### [Comparative Example 5]

A culture medium for evaluation was prepared in the same way as in Example 2 except that in this case no other substance was added to the D-MEM medium. By using the resulting culture medium for evaluation, the GPDH activity was measured in the same way as in Example 2.
The result of the measurement was 370 units.

When Example 2 (i.e. amino acid composition of the present invention) is compared with Comparative Example 3 (i.e. amino acid composition not conforming to the present invention), the values of the GPDH activity, which is an indicator of the inhibitory effect on differentiation to the adipocyte, are 3 units in Example 2, which means "excellent", and 168 units in Comparative Example 3, which means "poor", even though the same amounts of the amino acid composition (i.e. concentration of 1 mass% in the D-MEM medium) were used in Example 2 and Comparative Example 3. These values indicate a huge difference in effect between Example 2 and Comparative Example 3. As described above, the value of GPDH activity in Comparative Example 5, in which no amino acid composition is added, is 370 units.
A comparison of Example 2 (i.e. amino acid composition of the present invention) with Comparative Example 4 (i.e. adding Wortmannin) shows that "the amino acid composition of the present invention" (3 units) has a larger inhibitory effect on differentiation to the adipocyte than "with Wortmannin" (47 units) which is known to exhibit an inhibitory effect on differentiation to the adipocyte.

### [Experiments for Evaluation on the Fatty Acid Releasing Activity in Mouse Fibroblasts (Examples 3 to 5 and Comparative Examples 6 to 10)]

The induction of differentiation from mouse fibroblasts (3T3-L1 cells) to adipocytes was carried out in 12-well multiwell plates according to a known procedure. After treatment over about 2 weeks to induce differentiation to adipocytes, it was confirmed under a microscope that large differences in the differentiation induction activity were not present among these wells.

### [Example 3]

The culture medium was removed, and 500 µL of a phosphate buffer (PBS solution) that contained 0.2 mass% of an amino acid composition of the present invention (the same amino acid composition as in Example 1, also referred to as the "present amino acid composition" in this Description) was then added to each well. After 4 hours, 5 µL of the PBS solution was picked up and was transferred to a 96-well multiwell plate. The amount of free-fatty acid was measured on the 96-well multiwell plate by using a NEFA-C Test Wako which is for measuring free-fatty acid.
The principle of measuring the amount of free-fatty acid is as follows. AcylCoA is produced by the reaction between acylCoA synthetase (ACS) and free-fatty acid. The reaction of acylCoA oxidase (ACOD) with the produced acylCoA generates hydrogen peroxide, and in the presence of this hydrogen peroxide, oxidative condensation between 3-methyl-N-ethyl-N-(β-hydroxyethyl)aniline (MEHA) and 4-aminoantipyrine was done, and thereby a blue-purple dye was produced. The amount of free fatty acid can be detected by measurement of the optimal absorbance of this blue-purple dye. A large quantity of free fatty acid indicates a high level of lipolysis and an inhibition of intracellular fat accumulation in the mouse.
This experiment was carried out in triplicate and the average value and the standard deviation were calculated on these data.
The average value of absorbance was 0.083, and the standard deviation was 0.044.
An absorbance of 0.086 corresponds to 0.25 mEq/L of oleic acid.

### [Example 4]

An experiment was carried out in the same way as in Example 3 except that in this case the concentration of the amino acid composition was set to 0.04 mass%.
The average value of absorbance in Example 4 was 0.046, and the standard deviation was 0.037.

### [Example 5]

An experiment was carried out in the same way as in Example 3 except that in this case the concentration of the amino acid composition was set to 0.008 mass%.
The average value of absorbance in Example 5 was 0.049, and the standard deviation was 0.025.

### [Comparative Example 6]

An experiment was carried out in the same way as in Example 3 except that in this case the amino acid composition of the present invention in Example 3 was replaced by an amino acid composition prepared by mixing the branched-chain amino acids consisting of valine, leucine, and isoleucine (also referred to as "BCAA" in this Description) with the mass ratio of 1 : 2 : 1.
The average value of the absorbance was 0.082, and the standard deviation was 0.003.

### [Comparative Example 7]

An experiment was carried out in the same way as in Comparative Example 6 except that in this case the concentration of the amino acid composition was set to 0.04 mass%.
The average value of absorbance in Comparative Example 7 was 0.048, and the standard deviation was 0.022.

### [Comparative Example 8]

An experiment was carried out in the same way as in Comparative Example 6 except that in this case the concentration of the amino acid composition was set to 0.008 mass%.
The average value of absorbance in Comparative Example 8 was 0.038, and the standard deviation was 0.013.

### [Comparative Example 9]

An experiment was carried out in the same way as in Example 3 except that in this case the PBS solution containing the amino acid composition of the present invention was replaced by a PBS solution which contains 1 µM of dl-isoproterenol. "dl-isoproterenol" is a known compound that is known to exhibit a fatty acid releasing activity.
The average value of the absorbance was 0.041, and the standard deviation was 0.004.

### [Comparative Example 10]

An experiment as a control experiment was carried out in the same way as in Example 3 except that the PBS solution containing the amino acid composition of the present invention was replaced by the PBS solution without amino acid composition.
The average value of absorbance was 0.024, and the standard deviation was 0.022.

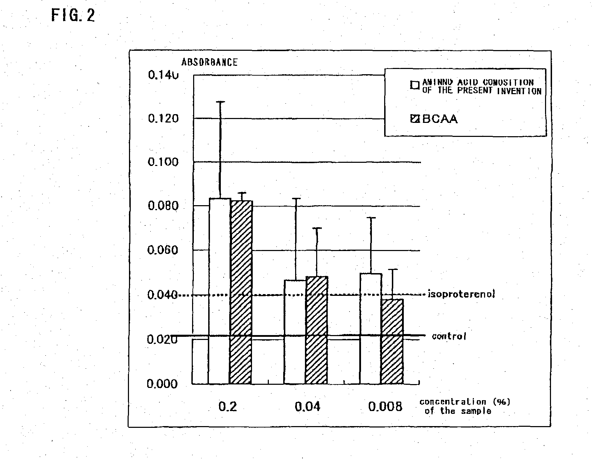

The preceding results (i.e. average value, and standard deviation) are shown in Figure 2.
Figure 2 shows that, at concentrations of 0.2 mass% and 0.04 mass% respectively in PBS solution (Examples 3 and 4), the amino acid composition of the present invention exhibits a fatty acid releasing activity to the same degree as BCAA. It is also shown that, at a concentration of 0.008 mass% in PBS solution (Example 5), the amino acid composition of the present invention exhibits a higher free-fatty acid releasing activity than the same concentration of BCAA.

### [Experiments for Evaluating the Effect of the Continuous Administration of the Amino Acid Composition on the Mouse Swimming Time (Examples 6 and Comparative Examples 11 and 12)]

Thirty male ddY mice (age: 4 weeks) were acclimated for 1 week. After that, the swimming time (i.e. period from the start of swimming to the physical limit of the mouse for swimming) of each mouse was determined. The thirty mice were then divided into 3 groups of ten mice each so as to provide the same average swimming time for each group.

### [Example 6]

At the time of one week after the grouping as described above, physiological saline containing the same amino acid composition as in Example 1 was orally administered to the mice so as to provide an amino acid composition administration of 500 mg per 1 kg of mouse body weight. The mouse swimming time (first) was measured at the time of 1 day after the administration. The mouse swimming time is the period from the start of swimming to the physical limit of the mouse for swimming (i.e. the time when the mouse was drowned) when a weight having 5 mass% of the body weight was attached to the tail of the mouse.
The result was a swimming time of 19.1 minutes (average value for 10 animals; this also applies in the following), and the standard error was 0.85 minute.
After that, the same amount of amino acid composition as described above was orally administered once per day continuously for 6 weeks. The swimming time was measured, at 1 week, 2 weeks, and 3 weeks after the first swimming test by using the same swimming test as the first swimming test. On the day when the swimming time was measured, the amino acid composition was orally administered 30 minutes before the start of swimming. An ordinary solid food was freely ingested during the experimental period.
The following results were obtained: after 1 week, the swimming time was 22.3 minutes and the standard error was 2.50 minutes; after 2 weeks, the swimming time was 26.7 minutes and the standard error was 3.41 minutes; after three weeks, the swimming time was 26.1 minutes and the standard error was 2.77 minutes.
After 6 weeks, at 30 minutes after the oral administration of the amino acid composition, a weight having 4.5 mass% of the body weight was attached to the tail of the mouse, and swimming was carried out for 15 minutes. During this experiment, the amount of 3-hydroxybutyric acid in the blood was measured just before oral administration, just before swimming (30 minutes after oral administration), and just after the completion of swimming. A large amount of 3-hydroxybutyric acid in the blood indicates a large amount of lipolysis and an inhibition of fat accumulation in the mouse.
The results for the amount of 3-hydroxybutyric acid in the blood were as follows: 31.2 µmol/L (standard error: 1.61 µmol/L) at just before oral administration; 33.2 µmol/L (standard error: 0.98 µmol/L) at just before swimming; and 37.5 µmol/L (standard error: 1.83 µmol/L) at just after the completion of swimming.

### [Comparative Example 11]

An experiment was carried out in the same way as in Example 6 except that in this case the amino acid composition used in Example 6 was replaced by an amino acid composition (BCAA) composed of mixing valine, leucine, and isoleucine with the mass ratio of 1 : 2 : 1.
The results were as follows: the swimming time in the first swimming test was 16.0 minutes (standard error: 1.03 minutes); the swimming time after 1 week was 16.0 minutes (standard error: 2.15 minutes); the swimming time after 2 weeks was 18.3 minutes (standard error: 2.65 minutes); and the swimming time after 3 weeks was 19.6 minutes (standard error: 2.74 minutes).
The amount of 3-hydroxybutyric acid in the blood was 33.3 µmol/L (standard error: 2.49 µmol/L) just before oral administration, 33.7 µmol/L (standard error: 1.84 µmol/L) just before swimming, and 37.6 µmol/L (standard error: 1.66 µmol/L) just after the completion of swimming.

### [Comparative Example 12]

An experiment was carried out in the same way as in Example 6 except that in this case the amino acid composition used in Example 6 was not used.
The results were as follows: the swimming time in the first swimming test was 15.4 minutes (standard error: 1.31 minutes); the swimming time after 1 week was 13.1 minutes (standard error: 1.62 minutes); the swimming time after 2 weeks was 15.1 minutes (standard error: 1.54 minutes); and the swimming time after 3 weeks was 15.5 minutes (standard error: 1.24 minutes).
The amount of 3-hydroxybutyric acid in the blood was 27.9 µmol/L (standard error: 1.25 µmol/L) just before oral administration, 26.9 µmol/L (standard error: 1.79 µmol/L) just before swimming, and 31.9 µmol/L (standard error: 1.53 µmol/L) just after the completion of swimming.

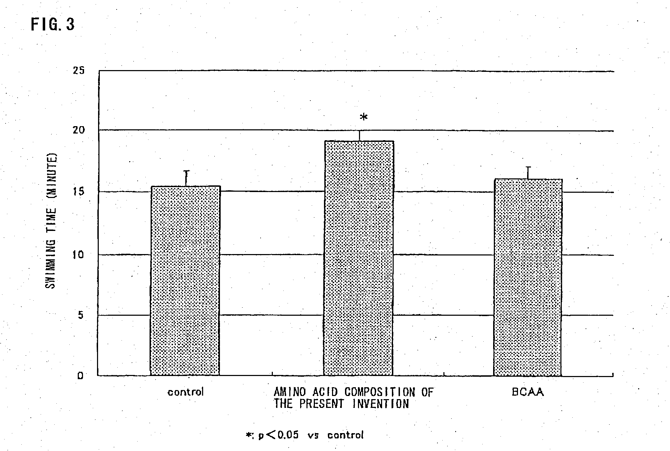

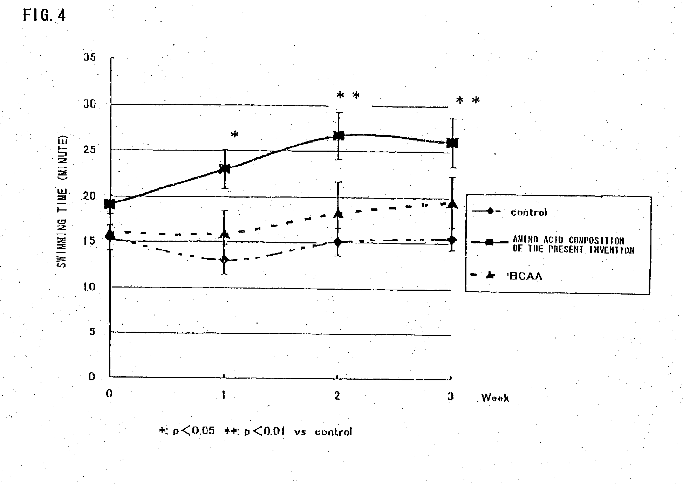

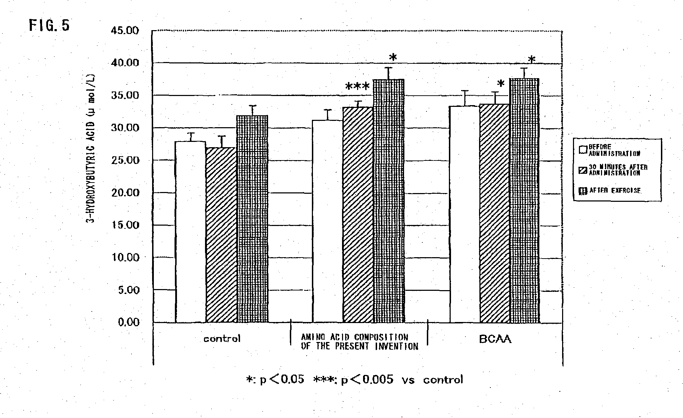

From among the results given above, the results for the first swimming time (i.e. average value, standard error) are shown in Figure 3. According to Figure 3, the use of the amino acid composition of the present invention (Example 6) provided a longer swimming time than the use of BCAA (Comparative Example 11) and a longer swimming time than the control group (Comparative Example 12).
The time course of the swimming time (i.e. average value, standard error) over the three weeks is shown in Figure 4. According to Figure 4, the case using the amino acid composition of the present invention (Example 6) provided a longer swimming time at each time point than the case using BCAA (Comparative Example 11) and the control group (Comparative Example 12).
The time course of the concentration of 3-hydroxybutyric acid in blood (i.e. average value, standard error) is shown in Figure 5. According to Figure 5, the case using the amino acid composition of the present invention (Example 6) provided a larger value than the control group (Comparative Example 12) at each time point, and provided about the same concentration of 3-hydroxybutyric acid in blood as for the case using BCAA (Comparative Example 11) at each time point.
No significant differences in body weight were seen during the experimental period among the mouse groups used in Examples and Comparative Examples.

### [Evaluation Tests of Resistance to Fatigue in the Mouse ; Example 7 and Comparative Example 13]

### [Example 7]

Physiological saline containing the same amino acid composition as in Example 1 was orally administered to mice so as to provide a dosage of 500 mg amino acid composition per 1 kg of mouse body weight. After forced running for 3 hours, the mice were held at quiescence for 15 minutes in a dark room. This was followed by the oral administration (second time) of the same amount of amino acid composition as in the first administration, and the spontaneous volume of exercise was automatically counted every 15 minutes over a 90 minute period in order to evaluate the anti-fatigue effect.
The spontaneous volume of exercise was counted using a "Supermex" (product name) from Muromachi Kikai Co., Ltd.
The anti-fatigue effect was also evaluated in the same way as described above except that the 3-hour forced running was not done (no-run group).
20 mice were in the forced running group, and 18 mice were in the no-run group.
As a result, the number of counts for the spontaneous exercise volume in the mice in the forced running group was an average of 149 (standard error: 33.4). The number of counts for the spontaneous exercise volume in the mice in the no-run group was an average of 182 (standard error: 42.8).
The difference between the counts (i.e. 182) in the no-run group and the counts (i.e. 149) in the forced running group was 33.

### [Comparative Example 13]

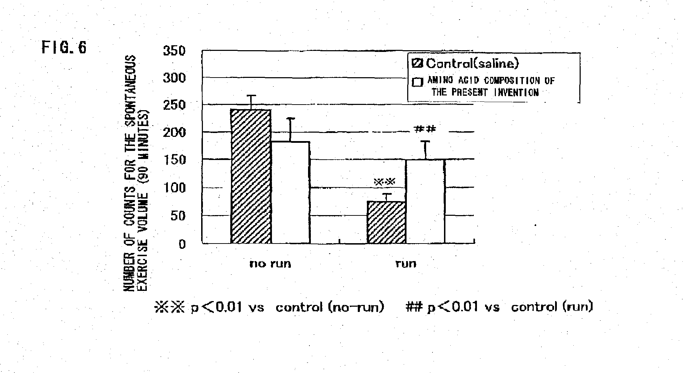

As a control example, an experiment was carried out in the same way as in Example 7 except that the amino acid composition was not used.
20 mice were in the forced running group, and 28 mice were in the no-run group.
As a result, the number of counts for the spontaneous exercise volume for the mice in the forced running group was an average of 74 (standard error: 13.9). The number of counts for the spontaneous exercise volume for the mice in the no-run group was an average of 241 (standard error: 25.9).
The difference between the counts (i.e. 241) in the no-run group and the counts (i.e. 74) in the forced running group was 167.
The preceding results are shown in Figure 6. In Figure 6, the no-run experiments are labeled with "no run" and the forced running experiments are labeled with "run". According to Figure 6, in the case that the physiological saline was used (Comparative Example 13), there was a large decline in the spontaneous exercise volume in forced running in comparison to no running, which demonstrated a high fatigue build-up in the mice due to the forced running. In contrast to this, in the case that the amino acid composition of the present invention was used (Example 7), there was little decline in the spontaneous exercise volume in forced running in comparison to no running, which indicated little fatigue build-up in the mice due to the forced running.

## Claims

1. An amino acid composition comprising the following amino acids in the following amounts wherein the total quantity of the following 18 amino acids is 100 mass parts.
| | |
|---|---|
| aspartic acid | 3.0 to 10.0 mass parts |
| threonine | 2.5 to 9.0 mass parts |
| serine | 2.0 to 9.5 mass parts |
| glutamic acid | 12.0 to 40.0 mass parts |
| proline | 7.0 to 20.0 mass parts |
| glycine | 0.5 to 4.5 mass parts |
| alanine | 1.0 to 4.5 mass parts |
| valine | 3.0 to 12.0 mass parts |
| cystine | 0.1 to 0.8 mass part |
| methionine | 1.5 to 5.0 mass parts |
| isoleucine | 2.0 to 8.0 mass parts |
| leucine | 5.0 to 15.0 mass parts |
| tyrosine | 3.0 to 10.0 mass parts |
| phenylalanine | 3.0 to 10.0 mass parts |
| lysine | 4.0 to 15.0 mass parts |
| tryptophan | 0.8 to 4.0 mass parts |
| histidine | 1.5 to 5.0 mass parts |
| arginine | 2.0 to 6.0 mass parts. |

2. The amino acid composition according to claim 1, wherein the amino acid composition is for oral ingestion.

3. The amino acid composition according to claim 1 or 2, wherein the amino acid composition is used to obtain at least one effect selected from promoting fat burning during exercise, improving endurance during exercise, preventing or reducing fatigue which is induced by exercise or supporting recovery from fatigue which is induced by exercise, obesity resistance, and stress resistance.
